# EUROPEAN PATENT SPECIFICATION

(11) **EP 4 385 475 B1**
(45) Date of publication and mention of the grant of the patent: **21.10.2026**
(21) Application number: 24173668.5
(22) Date of filing: 21.06.2019
(51) Int. Cl.: A61F 13/20

(54) **TAMPON FOR FEMININE HYGIENE**
TAMPON FÜR FRAUENHYGIENE
TAMPON POUR L'HYGIÈNE FÉMININE

(43) Date of publication of application: 19.06.2024
(62) Document number(s) of the earlier application(s) in accordance with Art. 76 EPC: 19733719.9 / 3 986 349
(73) Proprietor: Kenvue Germany GmbH, 41470 Neuss (DE)
(72) Inventor: BUSCHHAUS, Mirko, 45259 Essen (DE); LEYENDECKERS, Christian, 42289 Wuppertal (DE); ROBBE, Lionel, 42289 Wuppertal (DE); SPICHARTZ, Dorothea, 42289 Wuppertal (DE); WEINBERGER, Mike, 42289 Wuppertal (DE); WINKLER, Petra, 42289 Wuppertal (DE)
(74) Representative: Carpmaels & Ransford LLP

(56) References cited:
- WO-A1-2005/112856
- KR-B1- 101 760 825
- US-A1- 2007 049 893
- US-A1- 2014 052 090

## Description

### Field of the Invention

The present invention relates to an elongate absorbent tampon for feminine hygiene, more specifically to tampon with fluid transport elements, and a process for forming said tampon.

### BACKGROUND OF THE INVENTION

Devices for intravaginally capturing and storing bodily fluid are commercially available and known in the literature. Intravaginal tampons for feminine hygiene are the most common example of such devices. Commercially available tampons are generally compressed cylindrical masses of absorbent fibers that may be contained by an absorbent or nonabsorbent cover layer.

The tampon is inserted into the human vagina and retained there for a time for the purpose of capturing and storing intravaginal bodily fluids, most commonly menstrual fluid. As intravaginal bodily fluid contacts the tampon, it should be absorbed and retained by the absorbent material of the tampon. After a time, the tampon and its retained fluid is removed and disposed, and if necessary, another tampon is inserted.

A drawback often encountered with commercially available tampons is the tendency toward premature failure, which may be defined as bodily fluid leakage from the vagina while the tampon is in place, and before the tampon is completely saturated with the bodily fluid. The patent art typically describes a problem believed to occur that an unexpanded, compressed tampon is unable to immediately absorb fluid. Therefore, it presumes that premature leakage may occur when bodily fluid contacts a portion of the compressed tampon, and the fluid is not readily absorbed. The bodily fluid may bypass the tampon.

One way to prevent premature leakage from occurring is to provide designed pathways for fluid moving along the outer tampon surface. While this increase to the pathways may improve the fluid absorption, adding grooves during the manufacturing process can raise process issues. The prior art is replete with examples of attempts to incorporate grooves into tampons. Often new steps are added to an already complicated manufacturing process or the process is not fully described.

EP2712594B1 by JOHNSON & JOHNSON GmbH purports to disclose an intravaginal tampon for capturing menstrual fluid from human vagina, utilized for feminine hygiene application, having groove forms including turn unit proximate to insertion end and withdrawal end of cylindrical absorbent pledget.

EP2712595B1 by JOHNSON & JOHNSON GmbH purports to disclose an intravaginal tampon for feminine hygiene, the absorbent pledget is provided with continuous groove form comprising longitudinal groove segments linked to form turn located alternatively proximate to insertion and withdrawal ends.

EP2712596B1 by JOHNSON & JOHNSON GmbH purports to disclose a method for forming compressed tampon for feminine hygiene, said method involves extending pressing face of a first penetrating die beyond pressing face of a second die towards end of pledget, such that die pass through same space to form groove form.

WO2018220587 by JOHNSON & JOHNSON GmbH purports to disclose an apparatus for manufacturing an intravaginal tampon including a tampon press having a plurality of elongate press dies disposed about a central press axis to form a press cavity and a cylindrical carrier having a diameter less than that of the predetermined finished diameter of the intravaginal tampon. The elongate press dies include a plurality of longitudinal penetrating dies, each having a pressing face notch disposed proximate a first end thereof and an end notch disposed on an edge perpendicular to the pressing face at the opposite, second end thereof that cooperates with corresponding notches in an adjacent penetrating die.

Another way to overcome this problem of premature leakage, has been to incorporate extra elements into a basic tampon to try to direct and control the flow of fluid toward the absorbent core.

EP1748750B1 by JOHNSON & JOHNSON CONSUMER INC purports to disclose a fluid management device comprising fluid storage element and fluid transport element positioned within human vagina and comprising first and second plates for capturing bodily fluid in mammalian body.

EP1755514B1 by JOHNSON & JOHNSON CONSUMER COMPANIES, INC purports to disclose an intravaginal device having a fluid transport element with two plates, at least one of which has apertured film, and is bendable about axis parallel to longitudinal axis of fluid storage element, for capturing and storing body fluid.

EP1755515B1 by JOHNSON & JOHNSON CONSUMER INC purports to disclose a fluid management device for use in mammalian body that comprises fluid transport element providing uninterrupted fluid conduit to fluid storage element and comprising sheet-like first plate with outwardly and inwardly oriented surfaces
EP1758537B1 by JOHNSON & JOHNSON CONSUMER COMPANIES, INC purports to disclose an intravaginal device for capturing intravaginal bodily fluid, comprising fluid storage element and fluid transport element comprising flexible plate.

EP1765243B1 by JOHNSON & JOHNSON CONSUMER COMPANIES, INC purport to disclose an intravaginal device, e.g. tampon, for capturing and storing bodily fluid, that has bendable fluid transport element comprising two plates, and fluid storage element having longitudinal axis and in fluid communication with fluid transport element.

Yet another way to overcome this problem of premature leakage, has been to incorporate a channel from the insertion tip of a basic tampon to try to direct and control the flow of fluid toward the absorbent core.

Example of such prior art can be found in the tampon sold by DM under the brand Jessa in Germany and is called by the supplier a "safety channel". Said channels are narrower, typically 10% or less, than the tampon diameter.

While all these solutions provided by the prior art may reduce the risk of premature leakage, there is always a need from the consumers to improve the absorbent tampon construction to further decrease said leakage risk.

### BRIEF SUMMARY OF THE INVENTION

Surprisingly, we have found a novel way to address the problem of premature failure by combining two technologies that seem structurally incompatible. Tampons for feminine hygiene according to this invention have a compressed dome at the insertion end that expands even if covered by a fluid transport element that is attached to the longitudinal surfaces.

In our invention, we expand the insertion end of the tampon for quick absorption of large amount of fluid and deploy a fluid transport element to direct the fluid and minimize local saturation of the elongate tampon pledget. In our invention the insertion end of the tampon pledget is neat and aesthetically appealing and comfortable for the consumer. Our invention also is effective for handling highly viscous menstrual fluid.

In one aspect of the invention, an absorbent tampon for feminine hygiene includes a radially compressed, elongate tampon pledget and a fluid transport element. The elongate tampon pledget includes a domed insertion end, a withdrawal end comprising a withdrawal element, and a plurality of longitudinally extending grooves disposed along longitudinal surfaces of the elongate tampon pledget. At least a portion of the longitudinally extending grooves extend into the domed insertion end thereof. The fluid transport element includes an apertured polymeric film sheet disposed over the insertion end and attached to the elongate tampon pledget through a plurality of attachment zones extending along the longitudinal surfaces thereof. It also has a plurality of distal pleats to provide elements capable of extending away from the longitudinal surfaces of the elongate tampon pledget and a headspace between the domed insertion end and the apertured polymeric film sheet. A distance measured along an outer surface of the elongate tampon pledget over a longitudinal axis of the domed insertion end from an end of a first attachment zone nearest the domed insertion end to an end of a second attachment zone nearest the domed insertion end on an opposite surface defines a pledget dome length; a distance measured along an inner surface of the apertured polymeric film sheet over a longitudinal axis of the domed insertion end from the end of the first attachment zone nearest the domed insertion end to the end of a second attachment zone nearest the domed insertion end on the opposite surface defines a film length. The film length is greater than the pledget dome length.

In another aspect of the present invention, a process for forming an absorbent tampon for feminine hygiene includes radially compressing an elongate tampon blank, doming the insertion end of the elongate tampon pledget, and arranging a fluid transport element over the insertion end and attaching the fluid transport element to the elongate tampon pledget. The fluid transport element includes an apertured polymeric film sheet disposed over the insertion end and attached to the elongate tampon pledget through a plurality of attachment zones extending along the longitudinal surfaces thereof and further comprising a plurality of distal pleats to provide elements capable of extending away from the longitudinal surfaces of the elongate tampon pledget and a headspace between the domed insertion end and the apertured polymeric film sheet. A distance, measured along an outer surface of the elongate tampon pledget over a longitudinal axis of the domed insertion end, from an end of a first attachment zone nearest the domed insertion end, to an end of a second attachment zone nearest the domed insertion end, on an opposite surface defines a pledget dome length, and a distance, measured along an inner surface of the apertured polymeric film sheet over a longitudinal axis of the domed insertion end, from the end of the first attachment zone nearest the domed insertion end, to the end of a second attachment zone nearest the domed insertion end, on the opposite surface defines a film length. The film length is greater than the pledget dome length.

Other aspects and features of the present invention will become apparent in those ordinarily skilled in the art upon review of the following description of specific embodiments of the invention in conjunction with the accompanying drawings.

### BRIEF DESCRIPTION OF THE DRAWING

Fig. 1 is a side view of an absorbent tampon for feminine hygiene according to the present invention with outwardly extending fluid transport elements and a visible headspace.
Fig. 2. Is a side view of the absorbent tampon of Fig. 1 with fluid transport elements wrapped around the elongate tampon pledget.
Fig. 3 is a cross-section of the absorbent tampon of Fig. 2 along line A-A with outer wraps of the fluid transport element removed for clarity.
Fig. 4 is a side view of an absorbent tampon for feminine hygiene according to the present invention with fluid transport elements wrapped around the elongate tampon pledget and a portion of an apertured polymeric film sheet tucked into a cavity disposed in the insertion end of the elongate tampon pledget.
Fig. 5 is a cross-section of the absorbent tampon of Fig. 4 along line B-B.
Fig. 6 is an expanded view of a portion of two plates the fluid transport element between pleats of the apertured polymeric film sheet.
Fig. 7 is a cross section of an absorbent tampon for feminine hygiene deployed within a human vagina.
Fig. 8 is a side view of an elongate tampon pledget useful in the practice of the present invention.
Fig. 9 is a perspective view of a press useful in the method of the present invention with some elements removed for increased clarity of the illustrated press elements.
Fig. 10 is a perspective view of three of the press dies of the tampon press of the present invention including two penetrating dies and an intermediate shaping die, that together form a pair of penetrating groove segments that define a discrete surface zone.
Fig. 11 is a cross-section of the central portion of a press in an open position with outer portions broken away for increased clarity of the central press portion.
Fig. 12 is an enlarged cross-section view of the press during an initial compression step showing the penetrating die tips crossing into adjacent recesses.
Fig. 13 is a cross section of the press of Fig. 12 showing penetrating dies with crossing tips and shaping dies disposed therebetween.
Fig. 14 is a top view of an absorbent tampon as it would appear during use with initial fluid absorption and expansion of the domed insertion end thereof.
Fig. 15 is a top view of an absorbent tampon of the present invention showing a portion of the apertured polymeric film sheet tucked into a cavity in the domed insertion end of the elongate tampon pledget.
Fig. 16 is a schematic view of several steps in a process according to the present invention that creates and maintains a cavity in the insertion end of the elongate tampon pledget.

### DETAILED DESCRIPTION OF THE PREFERRED EMBODIMENTS

As used in the specification and the claims, the term "pledget" and variants thereof relate to a pad or a compress of absorbent material such as fibers designed to absorb bodily fluids.

As used herein in the specification and the claims, the term "bodily fluid" and variants thereof mean bodily exudates, especially liquids that are produced by, secreted by, emanate from, and/or discharged from a human body. In particular, menstrual fluid.

As used herein in the specification and the claims, the term "fluids" and variants thereof relate to liquids, and especially bodily fluids.

As used herein in the specification and the claims, the term "sheet" and variants thereof relates to a portion of something that is thin in comparison to its length and breadth.

As used herein in the specification and the claims, the term "parallel plate" and variants thereof relates to a system of at least two relatively parallel sheets that are capable of moving fluids through inter-plate capillary action. The individual "plates" in the system may be flexible and/or resilient in order to move within their environment. However, they may be maintained in a substantially facing relationship with relatively constant separation at least in a localized portion of their structure (as compared with their relative length and width). Thus, two sheets could be fluted, but if the flutes are "nested", the sheets would generally remain generally parallel in any given localized portion.

As used herein in the specification and the claims, the term "in fluid communication" and variants thereof relate to elements that are arranged and configured to allow fluid to move therebetween. The fluid movement may be by interfiber capillary movement, intrafiber capillary movement, osmotic pressure, interplate capillary action, mechanical channeling, and the like.

As used in the specification and the claims, the term "self-sustaining shape" and variants thereof relate to a tampon pledget that is compressed and/or shaped to assume a general shape and size that is dimensionally stable. For example, a digital tampon that has a self-sustaining shape will generally maintain its shape after a primary package or overwrap is removed and will generally maintain such shape for vaginal insertion. It will be recognized that the tampon is intended to absorb bodily fluids, and may substantially change shape during use as it absorbs such fluids.

As used herein the specification and the claims, the term "groove" and variants thereof relate to an indention into the surface of the tampon. For clarification, grooves may be "penetrating grooves", extending at least 0.7 mm (or 10% of the radius, whichever is greater) into the tampon. Regions between grooves may take the form of ribs.

As used herein the specification and the claims, the term "groove form" and variants thereof relates to a groove or combination of groove segments that are connected in a visibly identifiable manner to provide a unique detached feature at least on the surface of the tampon pledget.

As used herein the specification and the claims, the term "turn" and variants thereof relates to a portion of the groove form in which the groove and/or groove elements reverse(s) upon itself/themselves in a substantially U-shaped or a substantially V-shaped configuration. A "turn" can also have a generally linear extension from the intersection, such as a substantially Y-shaped configuration.

As used herein the specification and the claims, the term "major axis" and variants thereof relating to the groove form is defined by the shortest line connecting the most distant points of the groove form. Generally, this major axis will pass through at least one turn proximate to one end of the pledget.

As used herein the specification and the claims, the term "longitudinal axis" and variants thereof relate to an axis that runs from the insertion end to the withdrawal end substantially through the center of the tampon.

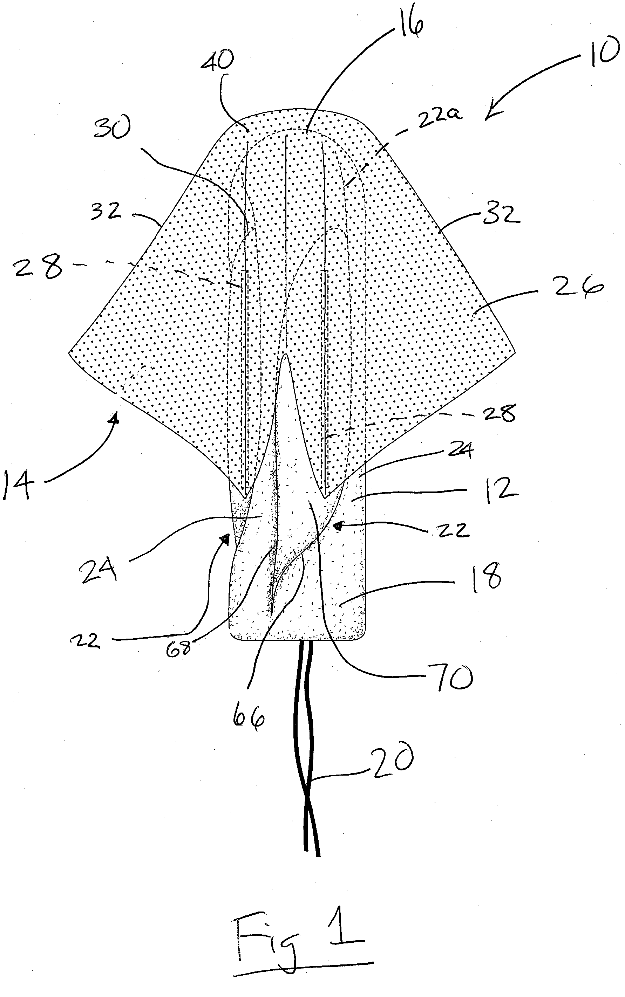

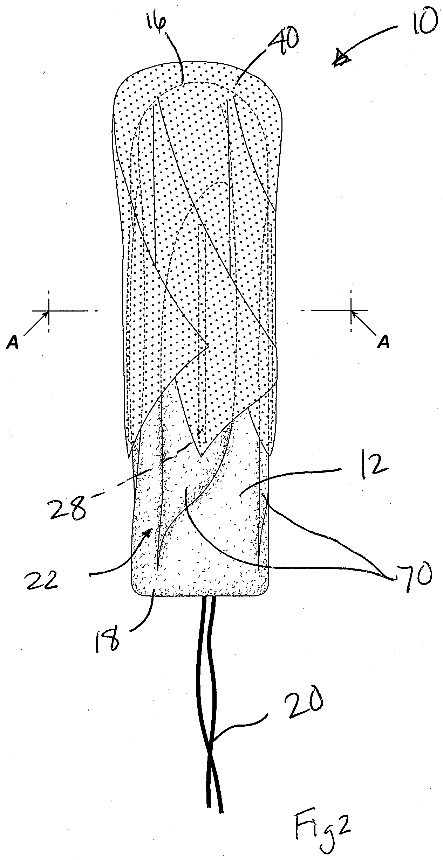

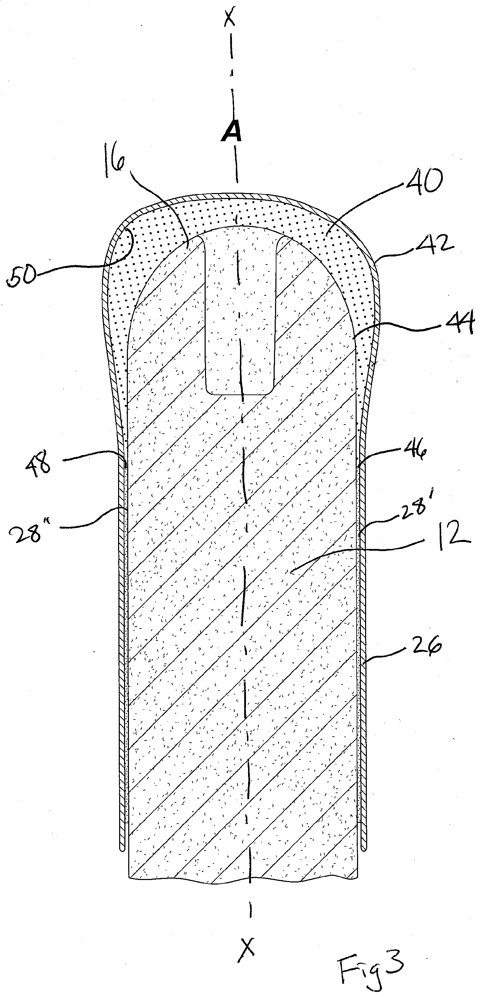

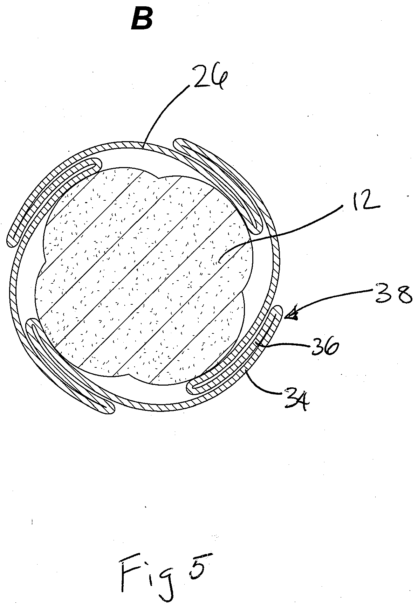

Referring to Figs. 1-3, the present invention provides an absorbent tampon 10 for feminine hygiene having a radially compressed, elongate tampon pledget 12 and a fluid transport element 14. The elongate tampon pledget 12 has a domed insertion end 16, a withdrawal end 18 comprising a withdrawal element 20, and a plurality of longitudinally extending grooves 22 disposed along longitudinal surfaces 24 of the elongate tampon pledget 12. At least a portion 22a of the longitudinally extending grooves 22 extend into the domed insertion end 16. The fluid transport element 14 is formed of an apertured polymeric film sheet 26 disposed over the insertion end 16 and attached to the elongate tampon pledget 12 through a plurality of attachment zones 28 extending along the longitudinal surfaces 24 thereof. The fluid transport element is folded to provide a plurality of proximal pleats 30 at least partially in the attachment zones 28 for securing the fluid transport element 14 to the elongate tampon pledget 12 and a plurality of distal pleats 32 to provide elements capable of extending away from the longitudinal surfaces 24 of the elongate tampon pledget 12 forming first and second plates 34, 36 that combine to provide sets of parallel plates 38 (see Fig. 5).

In addition, the attachment between the fluid transport element 14 and elongate tampon pledget 12 provides a headspace 40 between the domed insertion end 16 and the apertured polymeric film sheet 26. The headspace is provided by maintaining excess film sheet 42 centered over the domed insertion end 16. This can be described by (1) measuring a distance L_{PD} measured on an outer surface 44 of the elongate tampon pledget 12 over the longitudinal axis "x" of the domed insertion end 16 from an end 46 of a first attachment zone 28' nearest the domed insertion end 16 to an end 48 of a second attachment zone 28" nearest the domed insertion end 16 on an opposite surface which distance defines a pledget dome length; (2) measuring a distance L_{F} along an inner surface 50 of the apertured polymeric film sheet 26 over the longitudinal axis "x" of the domed insertion end 16 from the end 46 of a first attachment zone 28' nearest the domed insertion end 16 to the end 48 of a second attachment zone 28" nearest the domed insertion end 16 on the opposite surface defines a film length; and (3) maintaining a film length L_{F} greater than the pledge dome length L_{PD} to result in a positive value of a headspace length L_{H} (= L_{F} - L_{PD}).

### Fluid Transport Elements

The fluid transport elements include an apertured polymeric film sheet folded to form proximal pleats 30 and distal pleats 32. As these pleats bring two portions of the apertured polymeric film sheet in facing relationship, parallel plates 34, 36 are formed. The apertured polymeric film sheet forming the plates can be made of almost any hydrophobic or hydrophilic material, preferably sheet-like. The thickness of each plate is not critical. However, it can preferably be selected from the range of from about 0.005 to about 0.050 inch. The materials of construction and the thickness of the plates should be designed so that they are sufficiently stiff and/or resistant to wet collapse when exposed to fluid.

Materials useful for forming the fluid transport element may have properties such as thermobondability to provide means to incorporate it into the absorbent tampon. A representative, non-limiting list of useful materials includes polyolefins, such as polypropylene and polyethylene; polyolefin copolymers, such as ethylenevinyl acetate ("EVA"), ethylene-propylene, ethyleneacrylates, and ethylene-acrylic acid and salts thereof; halogenated polymers; polyesters and polyester copolymers; polyamides and polyamide copolymers; polyurethanes and polyurethane copolymers; polystyrenes and polystyrene copolymers; and the like. Examples of films having apertures include for example, three-dimensional apertured films, as disclosed in Thompson, U.S. Pat. No. 3,929,135, and Turi et al, U.S. Pat. No. 5,567,376, as well as two-dimensional reticulated film, such as that described in Kelly, U.S. Pat. No. 4,381,326.

It may be helpful to keep the exposed surface of the fluid transport element as smooth as possible. It may also be helpful to provide it with a low coefficient of friction. These characteristics may provide at least two benefits: (1) the force required to insert the absorbent tampon is reduced, and (2) it reduces the damage otherwise caused by scraping of soft, tender vaginal tissue during insertion, wearing and removal.

The parallel plates can have any physical structure to provide a resistance to fluid flow vector in the direction parallel to the inwardly oriented surface of the first plate and the first surface of the second plate that is less than the resistance to fluid flow vector in the direction perpendicular to the plates. Preferably, the plates are made from any relatively smooth material, such as apertured polymeric films.

The fluid transport element 14 should be strong enough to prevent rupturing during handling, insertion, and removal and to withstand vaginal pressures during use.

It is preferable that the surface of at least one of the plates of the fluid transport element 14 is sufficiently wettable by the bodily fluids that the absorbent tampon 10 is intended to collect (this results largely from a correlation of the surface energy of the plate surface and the bodily fluid(s)). Thus, the bodily fluid will easily wet the plate, and capillarity between the plates will draw these bodily fluids from a source to an elongate tampon pledget that is in fluid communication with the fluid transport element.

Surface treatments can be used to modify the surface energy of the plates 34, 36. In a preferred embodiment a surfactant is applied to increase the wettability of the outer or inner surfaces of the parallel plates. This will increase the rate at which the bodily fluids are drawn into and spread between a pair of plates. The surfactant can be applied uniformly to either the inner or outer surfaces or it could be applied with varying coating weights in different regions.

A useful measure to determine the wettability of a plate surface is its contact angle with 1.0% saline. Preferably, the contact angle with 1.0% saline is less than about 90 degrees.

In order to accomplish this, the materials of plates can be chosen from those materials that are known in the art to have low energy surfaces. It is also possible and useful to coat materials that have high-energy surfaces with a surface additive, such as a non-ionic surfactant (e.g., ethoxylates), a diol, or mixtures thereof, in order to increase their wettability by bodily fluids. Such additives are well known in the art, and examples include those described in Yang et al., US App. No. 2002-0123731-A1, and U.S. Pat. No. 6,570,055. Other means of increasing wettability can also be used, such as by corona discharge treatment of, for example, polyethylene or polypropylene, or by caustic etching of, for example, polyester.

The parallel plates forming the fluid transport element can be of any flexibility as long as the material is able to transport fluid to the elongate tampon pledget while the device is in use. It is preferable that the fluid transport element be sufficiently flexible to provide the user with comfort while inserting, wearing and removing the device.

Preferably, each plate has a plurality of openings distributed throughout. An example of openings distributed throughout is an apertured film. The distribution can be uniform or arranged to provide regions of higher open area and regions of lower open area. For example, a plurality of openings or apertures 52 may extend through at least one of the first and second plates 34, 36. These apertures 52 may extend completely through the plate and may be present in both of the plates. The apertures 52 allow fluid that contacts the outward surface of the first plate 34 or the opposite surface of the second plate 36 to flow into the inter-plate capillary gap 54 between the plates with as little restriction as possible. In the example of apertured film, it is preferred that the total surface area of the plate occupied by the openings is from about 5% to preferably about 50%. More preferably, it will be from about 25% to about 45%. Having this much open area formed in a plate will allow fluid that is deposited on that plate to easily flow into the inter-plate capillary gap 54.

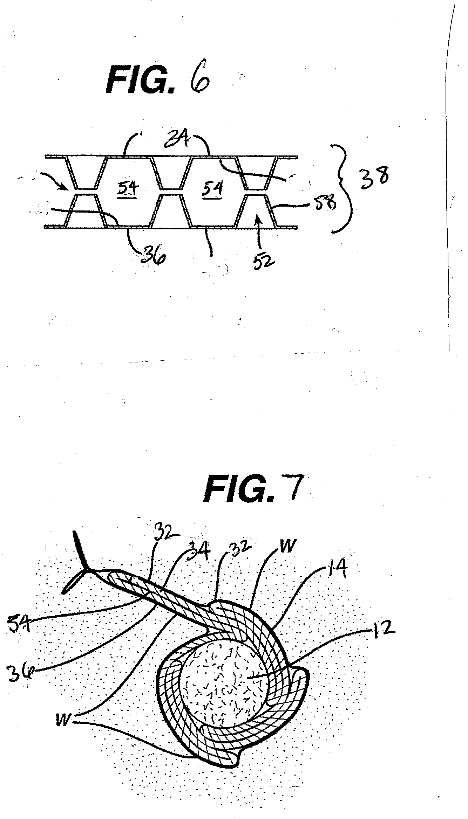

The plates are configured and arranged to allow the introduction of bodily fluids to separate a plate from adjacent plate(s). Optionally, one or more spacer elements can be inserted to establish and to maintain space between adjacent plates 34, 36. Spacer elements can be thickened portions of the plate material or deformations of the plate material. A representative list of such an integral spacer element includes, without limitation, nubbles, embossments, corrugations, deformations, and the like. Included in this definition are surface treatments that permanently bond a secondary material to a surface of a first. One example of a deformation is provided as the sidewalls 58 of a "three-dimensional" polymeric apertured formed film material shown in an enlarged view in Fig. 6. First and second plates 34, 36 made from apertured film with the sidewalls 58 facing each other as the inward surface of the first plate 34 and the inward surface of the second plate 36 can be used to increase the texture in order to break up the viscosity of the fluid being transported.

It is preferable that any individual opening is large enough to easily pass any highly viscous material, including menstrual fluid. While the geometry of the openings is not critical, the openings 52 should be sized sufficient to allow easy passage of non-absorbable material. If the apertures 52 are not circular, then the measurement should be made across the narrowest part of the opening, which would be most restrictive to the flow of non-absorbable material.

The first and second plates 34, 36 are extensions of the same sheet-like material, e.g., formed by a fold in a sheet of material. In such a folded embodiment, the material is folded to form a pleat with the first and second plates facing each other.

The fluid transport element 14 is in fluid communication with the elongate tampon pledget 12 and directs fluid from the vagina to the elongate tampon pledget 12. Generally, fluid will be directed from each fluid transport element 14 to a particular region of the elongate tampon pledget associated with that fluid transport element.

While the above description provides for direct fluid communication between a fluid transport element 14 and the elongate tampon pledget 12, direct fluid contact is not necessary. There can be fluid communication through an intermediate element, such as a porous medium (e.g., a foam or fibrous structure), a hollow tube, and the like.

The fluid transport element 14 may extend in any orientation from the surface of the elongate tampon pledget 12. It is not necessary for the fluid transport element to be on the surface of the elongate tampon pledget.

The fluid transport element 14 may be formed to extend from the surface of the elongate tampon pledget 12 as in FIGS. 1-7. It can be made in any convenient shape, including semicircular, triangular, square, hourglass etc. Additionally, the two plates of the element do not have to be completely coextensive, as long as they are at least partially in a facing relationship. In an alternative embodiment, the withdrawal string 20 could be replaced by a pair or another combination of ribbon-like parallel plates (not shown). Parallel plates can be held in close proximity to the elongate tampon pledget in a variety of ways including directly or indirectly via an additional element to the elongate tampon pledget. A variety of methods can be used to attach the fluid transport element 14 including but not limited to heat, adhesive, ultrasonics, sewing, and mechanically engaging the elongate tampon pledget 12.

During use, fluid transport element(s) 14 can take on many configurations within the vagina. For example, a distal pleat 32 fluid transport element 14 may extend into the vagina away from the elongate tampon pledget 12, as shown in FIG. 7. Alternatively, the fluid transport element(s) 14 may remain wound about the elongate tampon pledget 12, contacting the vaginal wall "W" only through the outwardly oriented surface.

### Elongate Tampon Pledget

The tampon of the present invention has reduced opportunity for bodily fluid to flow along the surface without being absorbed into the tampon pledget due to at least two detached groove forms each having a generally longitudinal orientation, a length (measured along the groove) that is at least 150% of the length of the pledget, and a turn proximate to at least one of an insertion end and a withdrawal end. The detached groove forms provide visually distinct zones with different bodily fluid handling characteristics. In addition, the turn proximate to at least one end of the tampon provides at least two groove paths for the fluid to follow to be distributed to different portions of the tampon pledget. Thus, not only does the present invention provide tampons with a plurality of grooves, recognized by the prior art as providing improved fluid handling characteristics, but it also provides either fully or partially closed absorption zones that visually communicate functional benefits to the user, including absorbent reservoirs to better contain bodily fluids in the tampon.

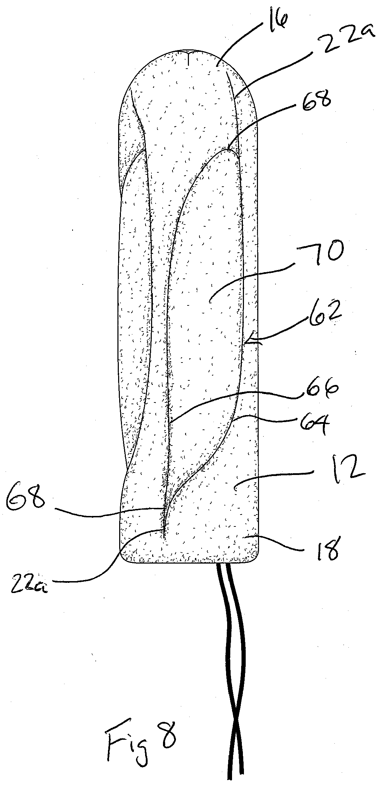

A preferred embodiment of the elongate tampon pledget 12 includes a plurality of detached groove forms 62 arranged about the outer surface of the elongate tampon pledget 12. In the embodiment of Fig. 8, the detached groove forms 62 each comprise a pair of groove segments 64, 68 that intersect to create a turn 68 proximate to both the insertion end 16 and the withdrawal end 18 of the elongate tampon pledget 12 to provide discrete surface zones 70 bounded by the encircling groove forms 62. In addition, a continuation 22a of one detached groove form extends beyond each turn 68.

The elongate absorbent pledget includes a mass of fibers compressed into a self-sustaining shape. The pledget may also include additional absorbent materials such as foam, superabsorbent, hydrogels, and the like. Preferred absorbent material for the present invention includes foam and fiber. Absorbent foams may include hydrophilic foams, foams which are readily wetted by aqueous fluids as well as foams in which the cell walls that form the foam themselves absorb fluid.

Preferably, the materials employed in the formation of a tampon according to the present invention include fiber, foam, hydrogels, wood pulp, superabsorbents, and the like. A useful, non-limiting list of useful absorbent body fibers includes natural fibers such as cotton, wood pulp, jute, and the like; and processed fibers such as regenerated cellulose, cellulose nitrate, cellulose acetate, rayon, polyester, polyvinyl alcohol, polyolefin, polyamine, polyamide, polyacrylonitrile, and the like. Other fibers in addition to the above fibers may be included to add desirable characteristics to the absorbent body. Preferably, tampon fibers are rayon, cotton, or blends thereof, and more preferably, the fibers are rayon. The fibers may have any useful cross-section.

Fiber cross-sections include multi-limbed and non-limbed. Multi-limbed, regenerated cellulosic fibers have been commercially available for a number of years. These fibers are known to possess increased specific absorbency over non-limbed fibers. A commercial example of these fibers is the Galaxy^{®} multilimbed viscose rayon fibers available from Kelheim Fibres GmbH, Kelheim, Germany. These fibers are described in detail in Wilkes et al., US Pat. No. 5,458,835, the disclosure of which is hereby incorporated by reference. Preferably, the fibers include hydrophilic fibers, and more preferably, the fibers include absorbent fibers, i.e., the individual fibers, themselves, absorb fluid. A non-limiting list of useful tampon fibers includes natural fibers such as cotton, wood pulp, jute, hemp, and the like; and processed fibers such as regenerated cellulose, cellulose nitrate, cellulose acetate, rayon, polyester, polyvinyl alcohol, polyolefin, polyamine, polyamide, polyacrylonitrile, and the like. Other fibers in addition to the above fibers may be included to add desirable characteristics to the absorbent body. For example, hydrophobic fibers may be used in outer surfaces of the tampon to reduce surface wetness and hydrophilic fibers may be used to increase the rate of fluid transport into and throughout the body.

The pledget includes a mass of fibers substantially enclosed by a sheet-like cover material fluid-permeable cover. Thus, the cover encloses a majority of the outer surface of the tampon. This may be achieved as disclosed in Friese, U.S. Patent No. 4,816,100, the disclosure of which is herein incorporated by reference. In addition, either or both ends of the tampon may be enclosed by the cover. Of course, for processing or other reasons, some portions of the surface of the tampon may be free of the cover. For example, the insertion end of the tampon and a portion of the cylindrical surface adjacent this end may be exposed, without the cover to allow the tampon to more readily accept fluids.

The cover can ease the insertion of the tampon into the body cavity and can reduce the possibility of fibers being separated from the tampon. Useful covers are known to those of ordinary skill in the art, and they are generally dimensionally stable with low elongation in both the machine and cross-direction. They may be selected from an outer layer of fibers which are fused together (such as by thermobonding), a nonwoven fabric, an apertured film, or the like. Preferably, the cover has a hydrophobic finish.

While liquid permeable covers are beneficial additions to radially-compressed tampons, their dimensional stability can produce some processing challenges. For example, radially compressing a cylindrical tampon blank having a dimensionally stable cover disposed about the cylindrical outer surface can result in cover wrinkles or loose cover extending from the outer surface of the compressed tampon pledget. Therefore, many processes involving radial compression of a tampon blank account for this by folding or tucking the cover material into grooves or folds that penetrate relatively deeply into the absorbent structure.

A process useful in the formation of an intravaginal tampon for feminine hygiene of the present invention with grooved zones begins with an open fibrous structure. The open structure may be a nonwoven fibrous web, a mass of randomly or substantially uniformly oriented fibers and optional materials, such as foams, or particles, and the like. This mass is then manipulated to form a tampon blank.

A nonwoven web useful in the present invention can be formed in any manner desired by the person of ordinary skill in the art. For example, fibers can be opened and/or blended by continuously metering them into a saw-tooth opener. The blended fibers can be transported, e.g., by air through a conduit to a carding station to form a fibrous web. Alternatively, a mass of substantially randomly oriented fibers can be formed by opening and/or blending them, transporting them, as above, to a station to form, e.g., a teabag-type tampon blank. Further processes may employ oriented fibers in a fibrous tow.

The tampon blank can be further processed to form a tampon. In a tampon forming process, a web can be formed into a narrow, fibrous sliver and convolutely wound to form a tampon blank. In addition, a liquid-permeable cover material can be wrapped around the tampon blank to substantially contain the fibrous absorbent portion of the tampon. It may be desired to process the fibrous sliver with selective needle-punching of the sliver as disclosed in US Pat. No. 7,845,055 to Kimball et al., the disclosure of which is herein incorporated by reference.

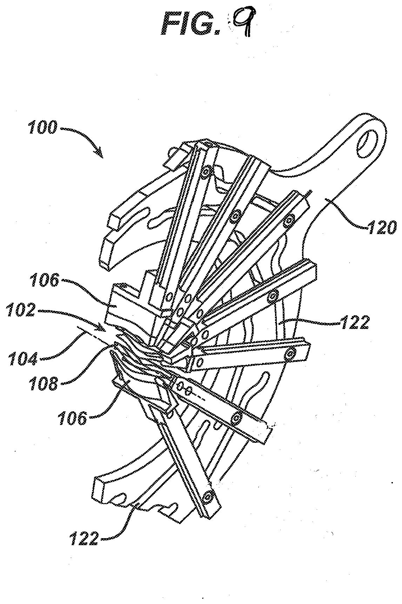

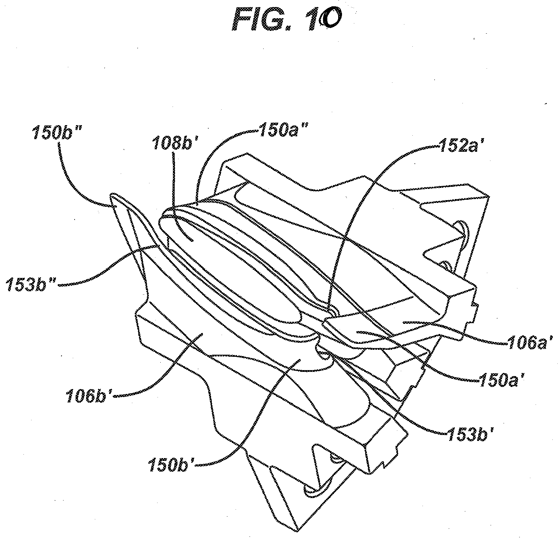

As shown in Fig. 9, the elongate tampon pledget for feminine hygiene of Fig. 8 having a predetermined finished diameter can be formed in a press 100 having (1) a generally cylindrical press cavity 102 having a central press axis 104 and a substantially cylindrical circumference and (2) a plurality of elongate press dies. A partially broken-away perspective view of the press 100 is shown in Fig. 10. This figure includes only seven of sixteen press dies and a portion of the press cam removed for clarity. The press dies may include penetrating dies 106 having pressing faces for defining a set of penetrating grooves that extend into the finished tampon pledget and shaping dies 108 for forming surface features, including shallow grooves on the outer surface of a resulting compressed tampon pledget, or smoothing the outer surface of a resulting compressed tampon pledget, or forming a continuous diameter for guiding resulting compressed tampon pledget out of the press during the ejection step. The penetrating dies 106 and shaping dies 108 alternate about the circumference of the cylindrical press cavity.

The press jaws of a preferred embodiment have a first penetrating die 106a having a pressing face 107 and shape corresponding to groove segment 64 and a second penetrating die 106b having a shape corresponding to groove segment 68 (of Fig. 8). As can be seen in Fig. 10, one end 150a of the first penetrating die 106a extends beyond the corresponding end 150b of the second penetrating die 106b. Indeed, the end 150b of the second penetrating die 106b is curved toward the first penetrating die 106a in order to form the turn 68 in the surface of the tampon elongate tampon pledget 12 (as shown in Fig. 8) proximate to the insertion end 16. In this embodiment, the end 150a of the first penetrating die 106a corresponds to the insertion end 16 of the tampon elongate tampon pledget 12 of Fig. 8.

Turn 68 of the detached groove form 62 is formed by the intersection between groove segments 64 and 66 (see Fig. 8). To form a groove form 62, the penetrating dies 106a,106b travel on a path that crosses during the compression of the tampon blank 200 (see Fig. 5) to form the elongate tampon pledget 12. Therefore, the longer penetrating die 106a has a notch 152 formed (see Fig. 10) proximate to, although spaced from, the end 150a to permit the end 150b of penetrating die 106b to pass across the path of travel of penetrating die 106a.

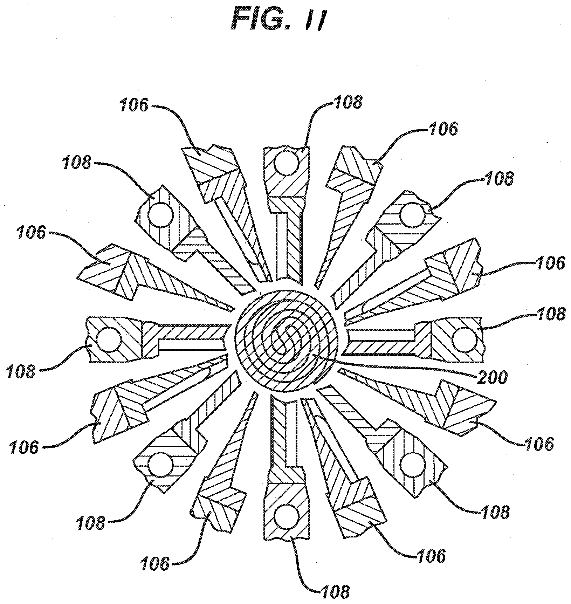

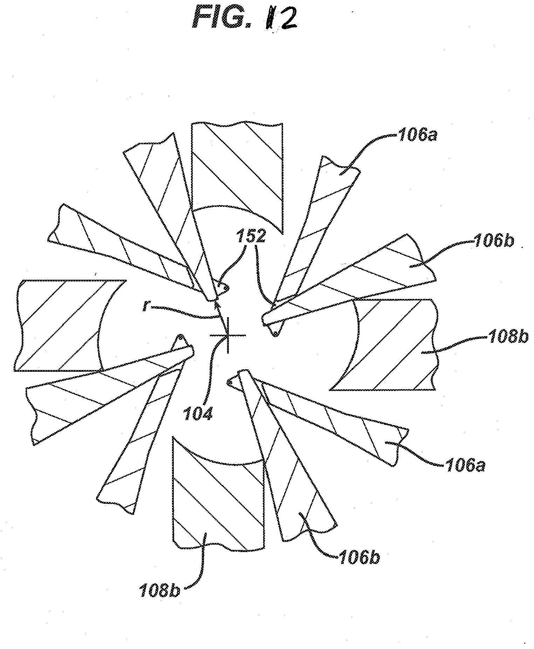

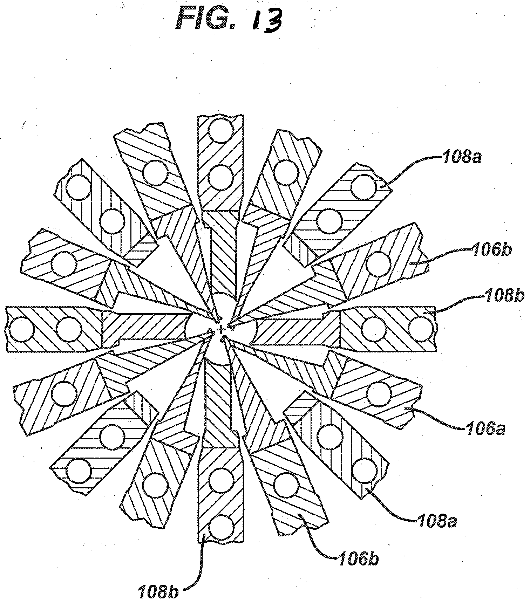

The shaping dies 108 are shaped to accommodate the shape of the penetrating dies 106 disposed therebetween. Thus, shaping die 108a corresponds to the surface of the elongate tampon pledget 12 contained by the groove segments 64 and 66 and the turn 68
In this process, a substantially cylindrical tampon blank 200 is inserted into the press cavity 102 in an open position shown in Fig. 11, after which an initial compression step is performed. In this initial compression step, at least the penetrating dies 106 are moved into the press cavity 102 to a penetrating die closed position having a clear distance "r" (see Fig. 12) from the press axis 104 that is less than the predetermined finished diameter as shown in Fig. 13. This causes portions of adjacent penetrating dies that form the turn to pass through the same space within the press. As shown in Fig. 10, this can be accomplished by forming a notch 152 in the first penetrating dies 106a to permit the second penetrating dies 106b to cross therethrough in the penetrating die closed position. This initial compression step forms the compressed fibrous core of the tampon and provides column strength for easy insertion without need for a tampon applicator, known in the art as digital insertion.

The tampon can be further shaped and packaged. For example, the insertion end can be formed into a hemispherical or elliptical dome shape, and the tampon can be enclosed in a primary packaging material that can also support the final shape of the tampon.

While the foregoing detailed embodiments describe tampons having four groove forms resulting from eight intersecting groove segments, it will be recognized that the number of groove forms and/or groove segments can be varied, as desired. There may be an even or odd number of groove forms and/or groove segments.

A withdrawal mechanism, such as withdrawal string 20, is preferably joined to the absorbent tampon 10 for removal after use. The withdrawal mechanism is preferably joined to at least the elongate tampon pledget 12 and extends beyond at least its withdrawal end 18. Any of the withdrawal strings currently known in the art may be used as a suitable withdrawal mechanism, including without limitation, braided (or twisted) cord, yarn, etc. In addition, the withdrawal mechanism can take on other forms such as a ribbon, loop, tab, or the like (including combinations of currently used mechanisms and these other forms). For example, several ribbons may be twisted or braided to provide parallel plates structures.

As mentioned above, the provision of sufficient "bagginess" or "headspace" - the gap between the domed insertion end 16 of the elongate tampon pledget 12 and the apertured polymeric film sheet 26 enables the domed insertion end 16 to expand similarly to the remainder of the elongate tampon pledget 12 upon absorption of bodily fluids. This contrasts with the tampons of, e.g., EP1755515B1 in which the fluid transport element closely follows the contours of the insertion end.

In a preferred embodiment shown in Fig. 1-3 the headspace length L_{H} is at least about 10 mm. Again, the headspace is determined by the difference in the distance between the film length L_{F} and the pledget dome length L_{PD}. The pledget dome length L_{PD} can be determined by measuring the distance along the outer surface 44 of the elongate tampon pledget 12 over the longitudinal axis "x" of the domed insertion end 16 from an end 46 of a first attachment zone 28' nearest the domed insertion end 16 to an end 48 of a second attachment zone 28" nearest the domed insertion end 16 on an opposite surface defines a pledget dome length. The measurement is obtained by averaging five measurements of a domed tampon insertion end using a flexible measuring tape in millimeters. The film length L_{F} can be determined by measuring the distance along the inner surface of the apertured polymeric film sheet from the same two attachment zone points (end 46 of a first attachment zone 28' and end 48 of a first attachment zone 28" after cutting the apertured polymeric film sheet at those two attachment zone points) and smoothing and laying the apertured polymeric film sheet under a thin clear holding plate, such as a 3 mm clear acrylic plate. Again, five measurements are averaged, and each measurement is taken after re-smoothing and laying the apertured polymeric film sheet under the thin clear holding plate.

In a preferred embodiment, the headspace length (L_{H} = L_{F} - L_{PD}) is at least about 10 mm. More preferably, the headspace length L_{H} is between about 10 mm and about 20 mm, and most preferably, the headspace length L_{H} is between about 12 mm and about 15 mm. Alternatively, the headspace may be expressed as the headspace ratio of: $\left({L}_{H}/diameter of the tampon\right) .$

Preferably, the headspace ratio is between about 0.6 and 1, and more preferably, the headspace ratio is between about 0.75 and about 0.95.

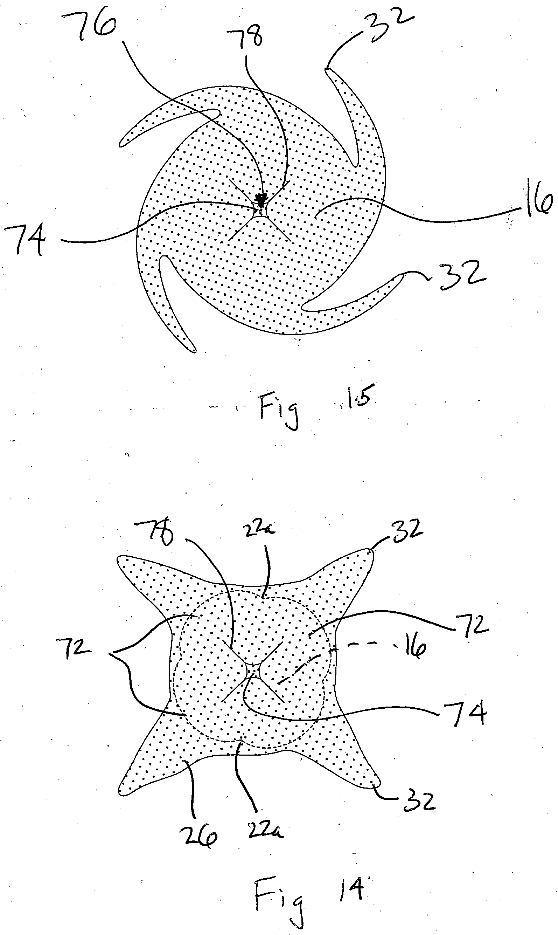

Another aspect of the present invention relates to locating the distal pleats 32 of the apertured film sheet 26 between the longitudinally extending grooves 22a in the domed insertion end 16. This aligns the distal pleats 32 with lobes 72 between the grooves 22a and allows these lobes 72 to expand more upon absorption of bodily fluids during use as shown in Fig. 14.

In a preferred embodiment, there are four distal pleats 32 aligned with the four lobes 72 that are disposed between four longitudinally extending grooves 22a in the domed insertion end 16.

The above arrangement can be achieved by forming intersecting, penetrating groove segments 64, 66 that define visually distinct, substantially closed zones 70 on the longitudinal surfaces thereof and locating the plurality of attachment zones 28 within these substantially closed zones 70.

In a preferred embodiment shown in Fig. 15, the domed insertion end 16 has a cavity 74 disposed therein, and a portion 76 of the apertured polymeric film sheet 26 is disposed that cavity 74 to present a smoother appearance. As the portion 76 of the apertured polymeric film sheet 26 is disposed into the cavity 74, some incidental folds 78 may develop in the apertured polymeric film sheet 26.

The method of producing absorbent tampons for feminine hygiene generally includes the steps of radially compressing an elongate tampon blank to form an elongate tampon pledget (each of the tampon blank and tampon pledget having corresponding insertion and withdrawal ends), doming the insertion end of the elongate tampon pledget, and arranging a fluid transport element over the insertion end of the elongate tampon pledget. The fluid transport element includes an apertured polymeric film sheet disposed over the insertion end and attached to the elongate tampon pledget through a plurality of attachment zones extending along the longitudinal surfaces thereof and further comprises a plurality of distal pleats to provide elements capable of extending away from the longitudinal surfaces of the elongate tampon pledget. A headspace is formed between the domed insertion end and the apertured polymeric film sheet by predetermined disposition of the apertured polymeric film sheet along the longitudinal surfaces of the elongate tampon pledget. Therefore, a distance, measured along an outer surface of the elongate tampon pledget over a longitudinal axis of the domed insertion end, from end of a first attachment zone nearest the domed insertion end, to end of a second attachment zone nearest the domed insertion end, on an opposite surface, defines a pledget dome length; and a distance, measured along an inner surface of the apertured polymeric film sheet over a longitudinal axis of the domed insertion end, from the end of the first attachment zone nearest the domed insertion end, to the end of a second attachment zone nearest the domed insertion end, on the opposite surface, defines a film length. The film length is greater than the pledget dome length, and this provides a headspace length that is greater than zero, as described above.

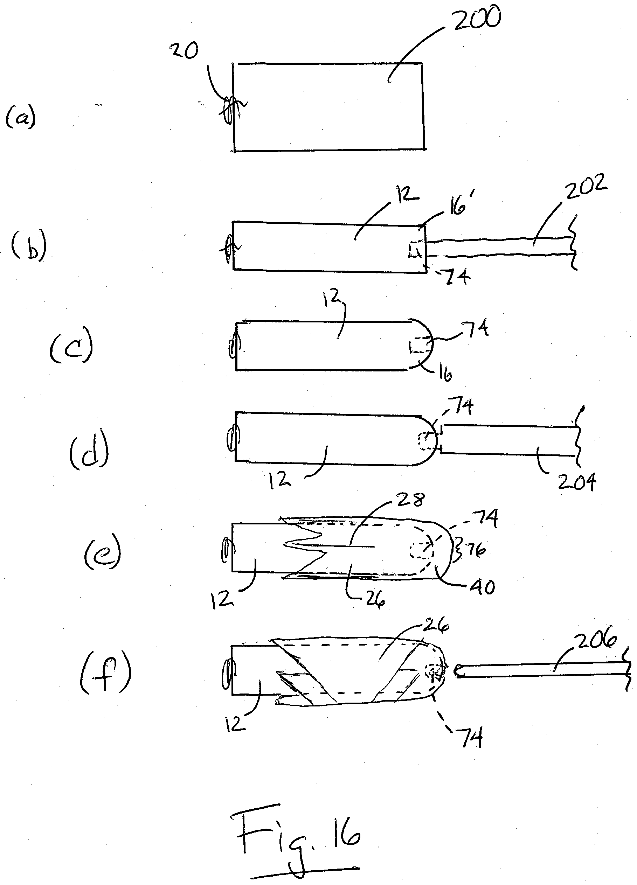

In a preferred embodiment of the method shown schematically in Fig. 16, a cavity 74 is formed in the domed insertion end 16 of the elongate tampon pledget 12. This provides a volume into which a portion 76 of the apertured polymeric film sheet 26 of the fluid transport element 14 may be tucked to present a smoother appearance to the insertion end 16 of the absorbent tampon 10. Step (a) shows a tampon blank 200 with a withdrawal element 20 wound up at one end, thereof, in preparation for radial compression in a tampon press. Step (b) shows the elongate tampon pledget 12 after radial compression in the tampon press and engagement of a pushrod 202 with the insertion end 16', and the initial formation of the cavity 74. The pushrod 202 has a cross-sectional diameter substantially less than a corresponding cross-sectional diameter of the elongate tampon pledget 12 and transfers the elongate tampon pledget 12 from the tampon press to a carrier (not shown) arranged and configured to contain the elongate tampon pledget 12 during transport for further processing. Step (c) shows the elongate tampon pledget 12 after doming the insertion end thereof to form the domed insertion end 16. The cavity 74 may be slightly closed from the doming operation, and therefore, the cavity 74 is refreshed or reformed using a tool 204 as shown in Step (d). Step (e) shows the fluid transport element 14 arranged and attached to the elongate tampon pledget 12 via attachment zones 28 and leaving a headspace 40 having a portion 76 of the apertured polymeric film sheet 26 located proximate the cavity 74. After the fluid transport element 14 is wound around the elongate tampon pledget 12, a finishing pushrod 206 places a portion 76 of the apertured polymeric film sheet 26 into the cavity 74.

After the fluid transport element 14 is attached to the longitudinal surfaces of the elongate tampon pledget 12, preferably by orienting an elongate tampon pledget 12 having longitudinally extending grooves 22 of the elongate tampon pledget 12 comprise intersecting, penetrating groove segments 64, 66 that define visually distinct, substantially closed zones 70 on the longitudinal surfaces thereof such that the attachment zones 28 for the fluid transport element 14 are disposed within the substantially closed zones 70, the portion 76 of the apertured polymeric film sheet 26 is inserted into the cavity 74 prior to packaging to smooth the domed insertion end 16 of the product 10.

Inserting and/or maintaining the portion 76 of the apertured polymeric film sheet 26 in the cavity can be performed before, during or after a further step of convolutely wrapping the distal pleats 32 of the fluid transport element 12 in a single direction around the elongate tampon pledget 12.

Another aspect of the invention that assists in allowing the domed insertion end 16 to more fully expand is to orient the distal pleats 32 with lobes 72 disposed between adjacent longitudinally extending grooves 22a in the domed insertion end 16 as shown in Fig. 14.

The cavity 74 formed preferably has a diameter and depth sufficient to accommodate the "excess" apertured polymeric sheet material 76. As a guide, in one embodiment, the cavity 74 has a maximum diameter of about 20% to about 50% of the maximum diameter of the tampon 10, more preferably about 25% to about 40% of the maximum diameter of the tampon 10 and a maximum depth of about 5 mm to about 10 mm. Both measurements are based on an average of five measurements of each element - the maximum cavity diameter and maximum tampon diameter are measured with a standard caliper without substantially deforming the fibrous structure and the maximum depth is measured with a pin having a diameter of approximately 50% of the diameter of the cavity.

Tampons are generally categorized in two classes: applicator tampons and digital tampons, and a certain amount of dimensional stability is useful for each type of tampon. Applicator tampons use a relatively rigid device to contain and protect the tampon prior to use. To insert the tampon into a body cavity, the applicator containing the tampon is partially inserted into the body cavity, and the tampon can be expelled from the applicator into the body cavity. In contrast, digital tampons do not have an applicator to help guide them into the body cavity and require sufficient column strength to allow insertion without using an applicator.

While the applicator tampon is protected by the rigid applicator device and the applicator tampon need not as have as high a degree of column strength as a digital tampon, applicator tampons do require dimensional stability (especially radial) to be acceptable for use. This dimensional stability provides assurance, for example, that the tampon will not prematurely grow and split its packaging material or become wedged in a tampon applicator.

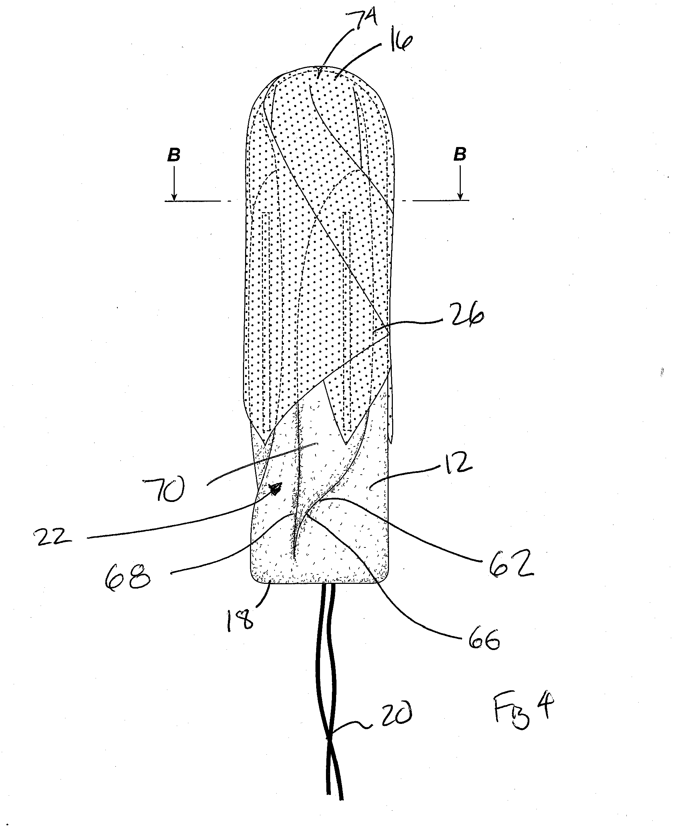

Further, the absorbent tampon can be collapsed for packaging and insertion. For example, at least a portion of a major surface of the fluid transport element 14, such as the outwardly oriented surface, may be in contact with at least a portion of an outer surface of the elongate tampon pledget 12. This can be achieved by wrapping the fluid transport element(s) around the elongate tampon pledget 12 (as shown in FIG. 4b). Alternatively, the fluid transport element(s) 14 may be folded or pleated (e.g., in an accordion-like manner) against the elongate tampon pledget 12.

The specification and embodiments above are presented to aid in the complete and non-limiting understanding of the invention disclosed herein. Since many variations and embodiments of the invention can be made without departing from its scope, the invention resides in the claims hereinafter appended.

## Claims

1. An absorbent tampon (10) for feminine hygiene comprising:
a) a radially compressed, elongate tampon pledget (12) having:
i) a domed insertion end (16);
ii) a withdrawal end (18) comprising a withdrawal element (20);
iii) a plurality of longitudinally extending grooves (22) disposed along longitudinal surfaces (24) of the elongate tampon pledget, at least a portion (22a) of the longitudinally extending grooves (22) extend into the domed insertion end; and
b) a fluid transport element (14) comprising an apertured polymeric film sheet (26) disposed over the insertion end and attached to the elongate tampon pledget through a plurality of attachment zones (28) extending along the longitudinal surfaces thereof and further comprising:
i) a plurality of distal pleats (32) to provide elements capable of extending away from the longitudinal surfaces of the elongate tampon pledget and
ii) a headspace (40) between the domed insertion end and the apertured polymeric film sheet;
wherein (A) a distance measured along an outer surface (44) of the elongate tampon pledget over a longitudinal axis of the domed insertion end from an end (46) of a first attachment zone (28') nearest the domed insertion end to an end (48) of a second attachment zone (28") nearest the domed insertion end on an opposite surface defines a pledget dome length; (B) a distance measured along an inner surface (50) of the apertured polymeric film sheet over a longitudinal axis of the domed insertion end from the end of the first attachment zone nearest the domed insertion end to the end of a second attachment zone nearest the domed insertion end on the opposite surface defines a film length; and (C) the film length is greater than the pledget dome length;
wherein domed insertion end has a cavity (74) formed therein and a portion (76) of the apertured polymeric film sheet is disposed in the cavity.

2. The absorbent tampon of claim 1 wherein the cavity has a diameter of about 20 to about 50% of a maximum corresponding diameter of the elongate tampon pledget, preferably about 25 to about 40%.

3. The absorbent tampon of claim 1 or 2 wherein the cavity has a depth of about 2 to about 15 mm, preferably about 5 to about 10 mm.

4. The absorbent tampon of any preceding claim wherein each distal pleat of the apertured polymeric film sheet is disposed between longitudinally extending grooves in the domed insertion end.

5. The absorbent tampon of claim 4 wherein the apertured polymeric film sheet has four distal pleats disposed between four longitudinally extending grooves in the domed insertion end.

6. The absorbent tampon of any preceding claim wherein the longitudinally extending grooves of the elongate tampon pledget comprises intersecting, penetrating groove segments (64, 66) that define visually distinct, substantially closed zones (70) on the longitudinal surfaces thereof and the plurality of attachment zones are disposed in the substantially closed zones.

7. A process for forming an absorbent tampon (10) for feminine hygiene of any of claims 1-6 comprising the steps of:
a) radially compressing a elongate tampon blank (200) to form a elongate tampon pledget (12) having a withdrawal end (18) and an insertion end corresponding to withdrawal end and an insertion end of the elongate tampon blank in a tampon press;
b) doming the insertion end of the elongate tampon pledget;
c) arranging a fluid transport element (14) over the insertion end and attaching the fluid transport element to the elongate tampon pledget through a plurality of attachment zones (28) extending along the longitudinal surfaces thereof (24), wherein
i) the fluid transport element comprises an apertured polymeric film sheet (26) disposed over the insertion end and attached to the elongate tampon pledget through a plurality of attachment zones extending along the longitudinal surfaces thereof and further comprising a plurality of distal pleats (32) to provide elements capable of extending away from the longitudinal surfaces of the elongate tampon pledget and a headspace (40) between the domed insertion end (16) and the apertured polymeric film sheet;
ii) a distance, measured along an outer surface (44) of the elongate tampon pledget over a longitudinal axis of the domed insertion end, from
a) an end (46) of a first attachment zone (28') nearest the domed insertion end, to
b) an end (48) of a second attachment zone (28") nearest the domed insertion end, on an opposite surface,
defines a pledget dome length;
iii) a distance, measured along an inner surface (50) of the apertured polymeric film sheet over a longitudinal axis of the domed insertion end, from
a) the end of the first attachment zone nearest the domed insertion end, to
b) the end of a second attachment zone nearest the domed insertion end, on the opposite surface,
defines a film length; and
iv) the film length is greater than the pledget dome length;
d) applying a pushrod (202) having a cross-sectional diameter substantially less than a corresponding cross-sectional diameter of the elongate tampon pledget to the insertion end of the elongate tampon pledget to transfer the elongate tampon pledget from the tampon press to a carrier arranged and configured to contain the elongate tampon pledget during transport for further processing, whereby a cavity (74) is formed in the insertion end of the elongate tampon pledget;
e) doming the insertion end of the elongate tampon pledget while maintaining the cavity in the insertion end of the elongate tampon pledget; and
f) inserting a portion (76) of the apertured polymeric film sheet into the cavity in the insertion end of the elongate tampon pledget.

8. The method of claim 7 further comprising the step of convolutely wrapping the distal pleats of the fluid transport element in a single direction around the elongate tampon pledget while applying pressure to the portion of the apertured polymeric film sheet disposed in the cavity.

9. The method of claim 7 or 8 further comprising the step of disposing each distal pleat of the apertured polymeric film sheet between adjacent longitudinally extending grooves in the domed insertion end.

10. The method of any of claims 7-9 wherein the longitudinally extending grooves of the elongate tampon pledget comprise intersecting, penetrating groove segments that define visually distinct, substantially closed zones on the longitudinal surfaces thereof and wherein the step of arranging the fluid transport element over the insertion end comprises aligning the plurality of attachment zones with the substantially closed zones.

## Patentansprüche

1. Saugfähiger Tampon (10) für die Damenhygiene, umfassend:
a) einen radial komprimierten, langgestreckten Tamponbausch (12) mit:
i) einem gewölbten Einführende (16);
ii) einem Rückholende (18), das ein Rückholelement (20) umfasst;
iii) mehreren in Längsrichtung verlaufenden Furchen (22), die entlang von Längsflächen (24) des langgestreckten Tamponbauschs angeordnet sind, wobei sich wenigstens ein Abschnitt (22a) der in Längsrichtung verlaufenden Furchen (22) in das gewölbte Einführende erstreckt; und
b) ein Fluidtransportelement (14), das eine mit Öffnungen versehene Polymerfolienlage (26) umfasst, die über dem Einführende angeordnet ist und durch mehrere Befestigungszonen (28), die sich entlang deren Längsflächen erstrecken, an dem langgestreckten Tamponbausch befestigt ist und ferner umfasst:
i) mehrere distale Falten (32), um Elemente bereitzustellen, die fähig sind, sich von den Längsflächen des langgestreckten Tamponbauschs weg zu erstrecken, und
ii) einen Kopfraum (40) zwischen dem gewölbten Einführende und der mit Öffnungen versehenen Polymerfolienlage;
wobei (A) ein Abstand, gemessen entlang einer Außenfläche (44) des langgestreckten Tamponbauschs über eine Längsachse des gewölbten Einführendes von einem Ende (46) einer ersten Befestigungszone (28'), das dem gewölbten Einführende am nächsten liegt, zu einem Ende (48) einer zweiten Befestigungszone (28'), das dem gewölbten Einführende am nächsten liegt, auf einer gegenüberliegenden Fläche, eine Bauschwölbungslänge definiert; (B) ein Abstand, gemessen entlang einer Innenfläche (50) der mit Öffnungen versehenen Polymerfolienlage über eine Längsachse des gewölbten Einführendes von dem Ende der ersten Befestigungszone, das dem gewölbten Einführende am nächsten liegt, zu dem Ende einer zweiten Befestigungszone, das dem gewölbten Einführende am nächsten liegt, auf der gegenüberliegenden Fläche, eine Folienlänge definiert; und (C) die Folienlänge größer als die Bauschwölbungslänge ist;
wobei das gewölbte Einführende einen darin gebildeten Hohlraum (74) aufweist und ein Abschnitt (76) der mit Öffnungen versehenen Polymerfolienlage in dem Hohlraum angeordnet ist.

2. Absorbierender Tampon nach Anspruch 1, wobei der Hohlraum einen Durchmesser von etwa 20 bis etwa 50 % eines maximalen entsprechenden Durchmessers des langgestreckten Tamponbauschs aufweist, vorzugsweise etwa 25 bis etwa 40 %.

3. Saugfähiger Tampon nach Anspruch 1 oder 2, wobei der Hohlraum eine Tiefe von etwa 2 bis etwa 15 mm, vorzugsweise etwa 5 bis etwa 10 mm, aufweist.

4. Absorbierender Tampon nach einem der vorstehenden Ansprüche, wobei jede distale Falte der mit Öffnungen versehenen Polymerfolienlage zwischen in Längsrichtung verlaufenden Furchen in dem gewölbten Einführende angeordnet ist.

5. Absorbierender Tampon nach Anspruch 4, wobei die mit Öffnungen versehene Polymerfolienlage vier distale Falten aufweist, die zwischen vier in Längsrichtung verlaufenden Furchen in dem gewölbten Einführende angeordnet sind.

6. Absorbierender Tampon nach einem der vorstehenden Ansprüche, wobei die in Längsrichtung verlaufenden Furchen des langgestreckten Tamponbauschs sich schneidende, durchdringende Furchensegmente (64, 66) umfassen, die visuell getrennte, im Wesentlichen geschlossene Zonen (70) auf ihren Längsflächen definieren, und die mehreren Befestigungszonen in den im Wesentlichen geschlossenen Zonen angeordnet sind.

7. Verfahren zum Bilden eines absorbierenden Tampons (10) für die Damenhygiene nach einem der Ansprüche 1-6, umfassend die Schritte:
a) radiales Komprimieren eines langgestreckten Tamponrohlings (200), um einen langgestreckten Tamponbausch (12) zu bilden, der ein Rückholende (18) und ein Einführende aufweist, die dem Rückholende und einem Einführende des langgestreckten Tamponrohlings entsprechen, in einer Tamponpresse;
b) Wölben des Einführendes des langgestreckten Tamponbauschs;
c) Anordnen eines Fluidtransportelements (14) über dem Einführende und Befestigen des Fluidtransportelements an dem langgestreckten Tamponbausch durch mehrere Befestigungszonen (28), die entlang der Längsflächen (24) davon verlaufen, wobei
i) das Fluidtransportelement eine mit Öffnungen versehene Polymerfolienlage (26) umfasst, die über dem Einführende angeordnet ist und durch mehrere Befestigungszonen, die entlang der Längsflächen davon verlaufen, an dem langgestreckten Tamponbausch befestigt ist, und ferner mehrere distale Falten (32) umfasst, um Elemente bereitzustellen, die sich von den Längsflächen des langgestreckten Tamponbauschs weg erstrecken können, und einen Kopfraum (40) zwischen dem gewölbten Einführende (16) und der mit Öffnungen versehenen Polymerfolienlage;
ii) ein Abstand, gemessen entlang einer Außenfläche (44) des langgestreckten Tamponbauschs über eine Längsachse des gewölbten Einführendes, von
a) einem Ende (46) einer ersten Befestigungszone (28'), das dem gewölbten Einführende am nächsten liegt, zu
b) einem Ende (48) einer zweiten Befestigungszone (28"), das dem gewölbten Einführende am nächsten liegt, auf einer gegenüberliegenden Oberfläche,
eine Bauschwölbungslänge definiert;
iii) ein Abstand, gemessen entlang einer Innenfläche (50) der mit Öffnungen versehenen Polymerfolienlage über eine Längsachse des gewölbten Einführendes, von
a) dem Ende der ersten Befestigungszone, das dem gewölbten Einsteckende am nächsten liegt, zu
b) dem Ende einer zweiten Befestigungszone, das dem gewölbten Einführende am nächsten liegt, auf der gegenüberliegenden Oberfläche,
eine Folienlänge definiert; und
iv) die Folienlänge größer ist als die Bauschwölbungslänge;
d) Anbringen eines Schubstabs (202) mit einem Querschnittsdurchmesser, der wesentlich kleiner als ein entsprechender Querschnittsdurchmesser des langgestreckten Tamponbauschs ist, an dem Einführende des langgestreckten Tamponbauschs, um den langgestreckten Tamponbausch von der Tamponpresse auf einen Träger zu überführen, der angeordnet und gestaltet ist, den langgestreckten Tamponbausch während des Transports zur weiteren Verarbeitung zu enthalten, wodurch ein Hohlraum (74) in dem Einführende des langgestreckten Tamponbauschs gebildet wird;
e) Wölben des Einführendes des langgestreckten Tamponbauschs unter Bewahrung des Hohlraums in dem Einführende des langgestreckten Tamponbauschs; und
f) Einführen eines Abschnitts (76) der mit Öffnungen versehenen Polymerfolienlage in den Hohlraum in dem Einführende des langgestreckten Tamponbauschs.

8. Verfahren nach Anspruch 7, ferner umfassend den Schritt des aufrollenden Wickelns der distalen Falten des Fluidtransportelements in einer einzigen Richtung um den langgestreckten Tamponbausch, während Druck auf den Abschnitt der in dem Hohlraum angeordneten mit Öffnungen versehenen Polymerfolienlage ausgeübt wird.

9. Verfahren nach Anspruch 7 oder 8, ferner umfassend den Schritt des Anordnens jeder distalen Falte der mit Öffnungen versehenen Polymerfolienlage zwischen benachbarten in Längsrichtung verlaufenden Furchen in dem gewölbten Einführende.

10. Verfahren nach einem der Ansprüche 7-9, wobei die in Längsrichtung verlaufenden Furchen des langgestreckten Tamponbauschs sich schneidende, durchdringende Furchensegmente umfassen, die visuell getrennte, im Wesentlichen geschlossene Zonen auf ihren Längsflächen definieren, und wobei der Schritt des Anordnens des Fluidtransportelements über dem Einführende Ausrichten der mehreren Befestigungszonen mit den im Wesentlichen geschlossenen Zonen umfasst.

## Revendications

1. Tampon absorbant (10) pour l'hygiène féminine comprenant :
a) une compresse de tampon allongée (12), comprimée radialement, ayant :
i) une extrémité d'insertion en forme de dôme (16) ;
ii) une extrémité de retrait (18) comprenant un élément de retrait (20) ;
iii) une pluralité de rainures s'étendant longitudinalement (22) disposées le long de surfaces longitudinales (24) de la compresse de tampon allongée, au moins une partie (22a) des rainures s'étendant longitudinalement (22) s'étendant dans l'extrémité d'insertion en forme de dôme ; et
b) un élément de transport de fluide (14) comprenant une feuille de film polymère perforée (26) disposée sur l'extrémité d'insertion et fixée à la compresse de tampon allongée par une pluralité de zones de fixation (28) s'étendant le long des surfaces longitudinales de celles-ci et comprenant en outre :
i) une pluralité de plis distaux (32) pour fournir des éléments capables de s'étendre à l'écart des surfaces longitudinales de la compresse de tampon allongée et
ii) un espace de tête (40) entre l'extrémité d'insertion en forme de dôme et la feuille de film polymère perforée, (A) une distance mesurée le long d'une surface extérieure (44) de la compresse de tampon allongée sur un axe longitudinal de l'extrémité d'insertion en forme de dôme depuis une extrémité (46) d'une première zone de fixation (28') la plus proche de l'extrémité d'insertion en forme de dôme jusqu'à une extrémité (48) d'une seconde zone de fixation (28") la plus proche de l'extrémité d'insertion en forme de dôme sur une surface opposée définissant une longueur de compresse en forme de dôme ; (B) une distance mesurée le long d'une surface intérieure (50) de la feuille de film polymère perforée sur un axe longitudinal de l'extrémité d'insertion en forme de dôme depuis l'extrémité de la première zone de fixation la plus proche de l'extrémité d'insertion en forme de dôme jusqu'à l'extrémité d'une seconde zone de fixation la plus proche de l'extrémité d'insertion en forme de dôme sur la surface opposée définissant une longueur de film ; et (C) la longueur de film étant supérieure à la longueur de compresse en forme de dôme, l'extrémité d'insertion en forme de dôme ayant une cavité (74) formée dans celle-ci, une partie (76) de la feuille de film polymère perforée étant disposée dans la cavité.

2. Tampon absorbant selon la revendication 1, la cavité ayant un diamètre d'environ 20 à environ 50 % d'un diamètre maximal correspondant de la compresse de tampon allongée, de préférence d'environ 25 à environ 40 %.

3. Tampon absorbant selon la revendication 1 ou 2, la cavité ayant une profondeur d'environ 2 à environ 15 mm, de préférence d'environ 5 à environ 10 mm.

4. Tampon absorbant selon l'une quelconque des revendications précédentes, chaque pli distal de la feuille de film polymère perforée étant disposé entre des rainures s'étendant longitudinalement dans l'extrémité d'insertion en forme de dôme.

5. Tampon absorbant selon la revendication 4, la feuille de film polymère perforée ayant quatre plis distaux disposés entre quatre rainures s'étendant longitudinalement dans l'extrémité d'insertion en forme de dôme.

6. Tampon absorbant selon l'une quelconque des revendications précédentes, les rainures s'étendant longitudinalement de la compresse de tampon allongée comprenant des segments de rainures pénétrantes qui se croisent (64, 66) qui définissent des zones visuellement distinctes, sensiblement fermées (70) les surfaces longitudinales de celles-ci, et la pluralité de zones de fixation étant disposées dans les zones sensiblement fermées.

7. Procédé de fabrication d'un tampon absorbant (10) pour l'hygiène féminine selon l'une quelconque des revendications 1 à 6 comprenant les étapes de :
a) compression radiale d'une ébauche de tampon allongée (200) pour former une compresse de tampon allongée (12) ayant une extrémité de retrait (18) et une extrémité d'insertion correspondant à l'extrémité de retrait et à une extrémité d'insertion de l'ébauche de tampon allongée dans une presse à tampon ;
b) bombage de l'extrémité d'insertion de la compresse de tampon allongée ;
c) agencement d'un élément de transport de fluide (14) sur l'extrémité d'insertion et fixation de l'élément de transport de fluide à la compresse de tampon allongée par une pluralité de zones de fixation (28) s'étendant le long des surfaces longitudinales de celles-ci (24),
i) l'élément de transport de fluide comprenant une feuille de film polymère perforée (26) disposée sur l'extrémité d'insertion et fixée à la compresse de tampon allongée par une pluralité de zones de fixation s'étendant le long des surfaces longitudinales de celles-ci et comprenant en outre une pluralité de plis distaux (32) pour fournir des éléments capables de s'étendre à l'écart des surfaces longitudinales de la compresse de tampon allongée et un espace de tête (40) entre l'extrémité d'insertion en forme de dôme (16) et la feuille de film polymère perforée,
ii) une distance, mesurée le long d'une surface extérieure (44) de la compresse de tampon allongée sur un axe longitudinal de l'extrémité d'insertion en forme de dôme, depuis
a) une extrémité (46) d'une première zone de fixation (28') la plus proche de l'extrémité d'insertion en forme de dôme, jusqu'à
b) une extrémité (48) d'une seconde zone de fixation (28") la plus proche de l'extrémité d'insertion en forme de dôme, sur une surface opposée,
définissant une longueur de compresse en forme de dôme,
iii) une distance, mesurée le long d'une surface intérieure (50) de la feuille de film polymère perforée sur un axe longitudinal de l'extrémité d'insertion en forme de dôme, depuis
a) l'extrémité de la première zone de fixation la plus proche de l'extrémité d'insertion en forme de dôme, jusqu'à
b) l'extrémité d'une seconde zone de fixation la plus proche de l'extrémité d'insertion en forme de dôme, sur la surface opposée,
définissant une longueur de film, et
iv) la longueur de film étant supérieure à la longueur de compresse en forme de dôme ;
d) application d'une tige de poussée (202) ayant un diamètre de section transversale sensiblement inférieur à un diamètre de section transversale correspondant de la compresse de tampon allongée à l'extrémité d'insertion de la compresse de tampon allongée pour transférer la compresse de tampon allongée depuis la presse à tampon à un support agencé et configuré pour contenir la compresse de tampon allongée pendant le transport en vue d'un traitement ultérieur, une cavité (74) étant ainsi formée dans l'extrémité d'insertion de la compresse de tampon allongée ;
e) bombage de l'extrémité d'insertion de la compresse de tampon allongée tout en maintenant la cavité dans l'extrémité d'insertion de la compresse de tampon allongée ; et
f) insertion d'une partie (76) de la feuille de film polymère perforée dans la cavité de l'extrémité d'insertion de la compresse de tampon allongée.

8. Procédé selon la revendication 7, comprenant en outre l'étape d'enroulement de manière convolutive des plis distaux de l'élément de transport de fluide dans une seule direction autour de la compresse de tampon allongée tout en appliquant une pression sur la partie de la feuille de film polymère perforée disposée dans la cavité.

9. Procédé selon la revendication 7 ou 8, comprenant en outre de disposition de chaque pli distal de la feuille de film polymère perforée entre des rainures adjacentes s'étendant longitudinalement dans l'extrémité d'insertion en forme de dôme.

10. Procédé selon l'une quelconque des revendications 7 à 9, les rainures s'étendant longitudinalement de la compresse de tampon allongée comprenant des segments de rainures pénétrantes qui se croisent qui définissent des zones visuellement distinctes, sensiblement fermées les surfaces longitudinales de celles-ci, et l'étape d'agencement de l'élément de transport de fluide sur l'extrémité d'insertion comprenant l'alignement de la pluralité de zones de fixation avec les zones sensiblement fermées.
